# EUROPEAN PATENT APPLICATION

(11) **EP 4 725 496 A2**
(43) Date of publication of application: **15.04.2026**
(21) Application number: 26161433.3
(22) Date of filing: 07.10.2021
(51) Int. Cl.: A61K 38/12

(54) **N-[4-[1-(1,4-DIOXASPIRO[4.5]DEC-8-YL)-4-(8-OXA-3-AZABICYCLO[3.2.1]OCT-3-YL)-1H-PYRAZOLO[3,4-D]PYRIMIDIN-6-YL]PHENYL]-N'-METHYLUREA FOR USE IN THE TREATMENT OF COVID19**

(30) Priority: 15.10.2020 US 202063092321 P
(62) Divisional of application: 21880808.7
(71) Applicant: NantCell, Inc., Culver City, CA 90232 (US)
(72) Inventor: BUZKO, Oleksandr, Culver City, 90232 (US); TANAKA, Shiho, Culver City, 90232 (US); NIAZI, Kayvan, Culver City, 90232 (US)
(74) Representative: J A Kemp LLP

(57) **Abstract**

The present disclosure relates to methods of treatment for COVID19.

## Description

### CROSS-REFERENCE TO RELATED APPLICATIONS

This application claims the benefit of priority under 35 U.S.C. §119(e) to U.S. Provisional Patent Application No. 63/092,321, filed October 15, 2020. The entire disclosure of U.S. Provisional Patent Application No. 63/092,321 is incorporated herein by reference.

### BACKGROUND

Coronavirus disease 2019 (COVID19) is the multisystem disease caused by the novel sudden acute respiratory syndrome coronavirus 2 (SARS-CoV-2). In a matter of months, COVID19 was recognized to be a significant health threat to the elderly and people with pre-existing medical conditions, and later as a significant health threat to everyone. Currently, there is no known cure for COVID19. Physicians, nurses, and scientists worldwide have undertaken an unprecedented, accelerated effort in finding treatments to ease COVID19 patient suffering, mitigate severity of disease symptoms, and prevent mortal outcomes.

COVID19 may affect every system in the human body, though it was first recognized by its effect on the respiratory system. SARS-CoV-2 is thought to initially infect a subject via the upper respiratory system, and the subsequent viral infection can be asymptomatic, mildly symptomatic, or severely symptomatic. Mild symptoms include fever (>100.4 °F), cough, fatigue, body aches, chills, sore throat, runny nose, congestion, conjunctivitis, gastrointestinal, abdominal pain, neurological including depression, tingling of extremities, and new loss of taste and smell. Severe symptoms and complications include shortness of breath (pneumonia), high fever, severe cough, seizures, stroke, blood clots, persistent chest pain/pressure, dizziness, delusion, confusion, inability to stay awake, acute respiratory distress syndrome, acute kidney injury, susceptible to additional infections (viral, bacterial, and fungal), heart problems, circulatory problems, sepsis, organ failure. Ultimately, COVID19 can cause death. A vast majority of recovered patients still experience persistent symptoms, including inflamed lungs, cardiac problems, chronic fatigue, and neurological issues months after their initial infection ("long-haulers"), even for patients with initially mild infections.

### SUMMARY

SARS-CoV-2 is an enveloped, non-segmented positive sense RNA virus of approximately 29.9 kb in length. The main structural proteins encoded by translation of the RNA sequence are the spike protein (S), small envelope protein (E), membrane glycoprotein (M), and nucleocapsid protein (N), along with several non-structural proteins (nsp 1 - 16) critical for new viral replication, construction, host cell response repression, and eventual exocytosis. The protease M^{pro}, or nsp5, plays an essential role in the virus life cycle. Nsp5 autoproteolyzes to become an active protease, and is responsible for the processing of active nsps 4 - 16. Additionally, nsp5 can potentially cleave host cell immune proteins (NLRP12, TAB1) and gene expression regulators (HDAC2, TRMT1).

The instant application provides methods for treating COVID19. Inhibiting the activity of nsp5 is an attractive target within the arsenal of weapons against SARS-CoV-2 infection and COVID19 progression. The instant application contains methods of treating COVID19 with compounds that target M^{pro} (nsp5).

In an aspect, provided herein, is a method of treating a SARS-CoV-2 virus infection in a subject in need thereof, the method including administering to the subject an effective amount of a pharmaceutical composition containing piperacillin or a salt thereof, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea (WYE-132) or a salt thereof, or cefoperazone or a salt thereof.

In an aspect, provided herein, is a method of treating a SARS-CoV-2 virus infection in a subject in need thereof, the method including administering to the subject an effective amount of a pharmaceutical composition containing cilengitide or a salt thereof, or hederacoside or a salt thereof.

In an aspect, provided herein, is a method of treating a SARS-CoV-2 virus infection in a subject in need thereof, the method including administering to the subject an effective amount of a pharmaceutical composition containing (R,S)-ivosidenib or a salt thereof, evacetrapib or a salt thereof, idasanutlin or a salt thereof, or relugolix or a salt thereof.

In embodiments, one or more additional compounds that treat SARS-CoV-2 virus infection, or symptom(s) thereof, are administered.

### BRIEF DESCRIPTION OF THE DRAWINGS

**FIG. 1** is a table of compounds that were tested as inhibitors of M^{pro}, or nsp5, protease activity. The observed activity of each compound against M^{pro} (nsp5) at 10 µM is indicated. Published IC50 values are provided, if known.
**FIG. 2** is a bar graph showing the percent M^{pro}, or nsp5, inhibitory activity per compound tested. The conditions for the FRET activity assay are based on Ma C, et al. Boceprevir, GC-376, and calpain inhibitors II, XII inhibit SARS-CoV-2 viral replication by targeting the viral main protease. Cell Research. (2020) 0:1-15.

### DETAILED DESCRIPTION

After reading this description, it will become apparent to one skilled in the art how to implement the present disclosure in various alternative embodiments and alternative applications. However, all the various embodiments of the present invention will not be described herein. It will be understood that the embodiments presented here are presented by way of an example only, and are not limiting. As such, this detailed description of various alternative embodiments should not be construed to limit the scope or breadth of the present disclosure as set forth herein.

Before the present technology is disclosed and described, it is to be understood that the aspects described below are not limited to specific compositions, methods of preparing such compositions, or uses thereof as such may, of course, vary. It is also to be understood that the terminology used herein is for the purpose of describing particular aspects only and is not intended to be limiting.

The detailed description divided into various sections only for the reader's convenience and disclosure found in any section may be combined with that in another section. Titles or subtitles may be used in the specification for the convenience of a reader, which are not intended to influence the scope of the present disclosure.

### Definitions

Unless defined otherwise, all technical and scientific terms used herein have the same meaning as commonly understood by one of ordinary skill in the art to which this disclosure belongs. In this specification and in the claims that follow, reference will be made to a number of terms that shall be defined to have the following meanings:

The terminology used herein is for the purpose of describing particular embodiments only and is not intended to be limiting. As used herein, the singular forms "a", "an" and "the" are intended to include the plural forms as well, unless the context clearly indicates otherwise.

"Optional" or "optionally" means that the subsequently described event or circumstance can or cannot occur, and that the description includes instances where the event or circumstance occurs and instances where it does not.

The term "about" when used before a numerical designation, *e.g*., temperature, time, amount, concentration, and such other, including a range, indicates approximations which may vary by ( + ) or ( - ) 10%, 5%, 1%, or any subrange or subvalue there between. Preferably, the term "about" when used with regard to an amount means that the amount may vary by +/- 10%.

"Comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the claimed invention. "Consisting of" shall mean excluding more than trace elements of other ingredients and substantial method steps. Embodiments defined by each of these transition terms are within the scope of this disclosure.

The term "coronavirus" refers to an RNA virus of the subfamily *Orthocoronviridae,* family *Coronaviridae,* order *Nidovirales,* and realm of *Riboviria.*

The term "SARS" or "Sudden Acute Respiratory Syndrome" refers to a disease caused by a SARS-coronavirus.

The term "SARS-CoV-2" or "Sudden Acute Respiratory Syndrome Coronavirus 2" or "2019-nCoV" refers to the coronavirus causative of COVID19. SARS-CoV-2 is a lipid-enveloped, positive-sense, single-strand RNA virus of the genus *Betacoronavirus* of the *Orthocoronaviridae* subfamily of coronaviruses.

The term "COVID19" or "COVID-19" or "Coronavirus Disease 2019" refers to the disease caused by an SARS-CoV-2 infection in a subject.

The term "M^{pro}" or "Mpro" or "3CL^{pro}" or "C3-like main protease" or "main protease" or "nsp5 protease" refers to a trypsin/chymotrypsin-like, and picornavirus 3C-like protease that processes the SARS viral polyproteins. M^{pro}, or nsp5, also processes several host cell proteins, including removing ubiquitin from ubiquintinated proteins.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

One skilled in the art would understand that descriptions of making and using the complexes described herein is for the sole purpose of illustration, and that the present disclosure is not limited by this illustration.

The publications discussed herein are provided solely for their disclosure prior to the filing date of the present application. Nothing herein is to be construed as an admission that such publications constitute prior art to the claims appended hereto. All publications referenced herein are incorporated herein by reference in their entireties for all of their teachings, including, but not limited to, all compositions, components, reagents, and methods.

### COVID Treatment Methods

In an aspect, provided herein, is a method of treating a SARS-CoV-2 virus infection in a subject in need thereof. In embodiments, the method includes administering to the subject an effective amount of a pharmaceutical composition containing piperacillin or a salt thereof, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof, and cefoperazone or a salt thereof. In embodiments, piperacillin or a salt thereof is administered. In embodiments, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof is administered. In embodiments, cefoperazone or a salt thereof is administered.

In embodiments, the piperacillin or salt thereof is administered in the amount of about 2 grams (g) to about 40 g. In some embodiments, the piperacillin or salt thereof is administered in the amount of about 2 g. In some embodiments, the piperacillin or salt thereof is administered in the amount of about 3 g. In some embodiments, the piperacillin or salt thereof is administered in the amount of about 4 g. In some embodiments, the piperacillin or salt thereof is administered in the amount of about 40 g. The amount may be any value or subrange within recited ranges, including endpoints. In some embodiments, the piperacillin or salt thereof is administered via intravenous injection. In some embodiments, the piperacillin or salt thereof is administered via intramuscular injection.

In embodiments, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 milligrams per kilogram body weight (mg/kg) to about 50 mg/kg. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 mg/kg. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 50 mg/kg. The amount may be any value or subrange within recited ranges, including endpoints. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered via intravenous injection. In some embodiments, the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered via intraperitoneal injection.

In embodiments, the method of treatment wherein the cefoperazone or salt thereof is administered in the amount of about 1 g to about 12 g per day (g/day). In some embodiments, the cefoperazone or salt thereof is administered in the amount of about 1 g/day. In some embodiments, the cefoperazone or salt thereof is administered in the amount of about 2 g/day. In some embodiments, the cefoperazone or salt thereof is administered in the amount of about 3 g/day. In some embodiments, the cefoperazone or salt thereof is administered in the amount of about 4 g/day. In some embodiments, the cefoperazone or salt thereof is administered in the amount of about 6 g/day. In some embodiments, the cefoperazone or salt thereof is administered in the amount of about 8 g/day. In some embodiments, the cefoperazone or salt thereof is administered in the amount of about 10 g/day. In some embodiments, the cefoperazone or salt thereof is administered in the amount of about 12 g/day. The amount may be any value or subrange within recited ranges, including endpoints. In some embodiments, the cefoperazone or salt thereof is administered via intravenous injection. In some embodiments, the cefoperazone or salt thereof is administered via subcutaneous injection.

In embodiments, one or more additional agents may be administered to the subject. The one or more additional agents may treat SARS-CoV-2 infection, and/or a symptom of SARS-CoV-2 infection. In embodiments, the one or more agents may include convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines. In embodiments, one or more of the recited agents may be expressly excluded.

In an aspect, provided herein, is a method of treating a SARS-CoV-2 virus infection in a subject in need thereof, the method including administering to the subject an effective amount of a pharmaceutical composition containing cilengitide or a salt thereof or hederacoside or a salt thereof. In embodiments, cilengitide or a salt thereof is administered. In embodiments, hederacoside or a salt thereof is administered.

In embodiments, the method of treatment, wherein the cilengitide or salt thereof is administered in the amount of about 30 milligrams per m² of body surface area (mg/ m²) to about 2400 mg/m². In some embodiments, cilengitide or salt thereof is administered in the amount of about 30 mg/m². In some embodiments, the method of treatment, wherein the cilengitide or salt thereof is administered in the amount of about 60 mg/m². In some embodiments, the method of treatment, wherein the cilengitide or salt thereof is administered in the amount of about 90 mg/m². In some embodiments, the method of treatment, wherein the cilengitide or salt thereof is administered in the amount of about 120 mg/m². In some embodiments, the method of treatment, wherein the cilengitide or salt thereof is administered in the amount of about 240 mg/m². In some embodiments, the method of treatment, wherein the cilengitide or salt thereof is administered in the amount of about 360 mg/m². In some embodiments, the method of treatment, wherein the cilengitide or salt thereof is administered in the amount of about 480 mg/m². In some embodiments, the method of treatment, wherein the cilengitide or salt thereof is administered in the amount of about 600 mg/m². In some embodiments, the method of treatment, wherein the cilengitide or salt thereof is administered in the amount of about 1200 mg/m². In some embodiments, the method of treatment, wherein the cilengitide or salt thereof is administered in the amount of about 1800 mg/m². In some embodiments, the method of treatment, wherein the cilengitide or salt thereof is administered in the amount of about 2400 mg/m². The amount may be any value or subrange within recited ranges, including endpoints. In some embodiments, the cilengitide or salt thereof is administered via intravenous injection. In some embodiments, the cilengitide or salt thereof is administered via intravenous infusion. In some embodiments, the cilengitide or salt thereof is administered via continuous intravenous infusion. In some embodiments, the cilengitide or salt thereof is administered via intraperitoneal injection.

In embodiments, hederacoside or salt thereof is administered in the amount of about 35 mg/kg to about 50 mg/kg. In some embodiments, hederacoside or salt thereof is administered in a 5 mL dose at a concentration of about 35 mg dry leaves in about 30% ethanol extract (5-7.5:1 DER). In some embodiments, hederacoside or salt thereof is administered in a 7.5 mL dose in a concentration of about 35 mg dry leaves in about 30% ethanol extract (5-7.5:1 DER). In some embodiments, wherein the hederacoside or salt thereof is administered at about 24 drops/dose of about 35 mg/dry leaves/30% ethanol extract (5-7.5:1 DER). In some embodiments, wherein the hederacoside or salt thereof is administered via 2.5 mL to 7.5 mL/dose syrup containing about 7 mg/mL of 43 mg of dry leaves in about 30% ethanol extract (5-7.5:1 DER). In some embodiments, wherein the hederacoside or salt thereof is administered in about 5 mL/dose syrup containing about 7 mg/mL of 43 mg dry leaves in about 30% ethanol extract (5-7.5:1 DER). In some embodiments, wherein the hederacoside or salt thereof is administered via chewable lozenge containing about 26 mg equivalent to about 163 mg dry leaf. The amount may be any value or subrange within recited ranges, including endpoints.

In an aspect, provided herein, is a method of treating a SARS-CoV-2 virus infection in a subject in need thereof, the method including administering to the subject an effective amount of a pharmaceutical composition containing (R,S)-ivosidenib or a salt thereof, evacetrapib or a salt thereof, idasanutlin or a salt thereof, or relugolix or a salt thereof. In embodiments, the pharmaceutical composition contains (R,S)-ivosidenib or a salt thereof. In embodiments, the pharmaceutical composition contains evacetrapib or a salt thereof. In embodiments, the pharmaceutical composition contains idasanutlin or a salt thereof. In embodiments, the pharmaceutical composition contains relugolix or a salt thereof.

In embodiments, evacetrapib or salt thereof is administered in an amount of about 30 mg to about 600 mg per day. In some embodiments, the evacetrapib or salt thereof is administered in an amount of about 30 mg/day. In some embodiments, the evacetrapib or salt thereof is administered in the amount of about 100 mg/day. In some embodiments, the evacetrapib or salt thereof is administered in the amount of about 130 mg/day. In some embodiments, the evacetrapib or salt thereof is administered in the amount of about 500 mg/day. In some embodiments, the evacetrapib or salt thereof is administered in the amount of about 600 mg/day. The amount may be any value or subrange within recited ranges, including endpoints. In some embodiments, the evacetrapib or salt thereof is administered orally. In some embodiments, the evacetrapib or salt thereof is administered via intravenous injection.

In embodiments, (R,S)-ivosidenib or salt thereof is administered in an amount of about 100 mg/day to about 1200 mg/day. In some embodiments, the (R,S)-ivosidenib or salt thereof is administered in the amount of about 100 mg/day. In some embodiments, the (R,S)-ivosidenib or salt thereof is administered in the amount of about 200 mg/day. In some embodiments, the (R,S)-ivosidenib or salt thereof is administered in the amount of about 300 mg/day. In some embodiments, the (R,S)-ivosidenib or salt thereof is administered in the amount of about 400 mg/day. In some embodiments, the (R,S)-ivosidenib or salt thereof is administered in the amount of about 500 mg/day. In some embodiments, the (R,S)-ivosidenib or salt thereof is administered in the amount of about 800 mg/day. In some embodiments, the (R,S)-ivosidenib or salt thereof is administered in the amount of about 1200 mg/day. The amount may be any value or subrange within recited ranges, including endpoints. In some embodiments, the (R,S)-ivosidenib or salt thereof is administered orally.

In embodiments, idasanutlin or salt thereof is administered in an amount of about 100 mg/day to about 300 mg/day. In some embodiments, the idasanutlin or salt thereof is administered in the amount of about 100 mg/day. In some embodiments, the idasanutlin or salt thereof is administered in the amount of about 120 mg/day. In some embodiments, the idasanutlin or salt thereof is administered in the amount of about 150 mg/day. In some embodiments, the idasanutlin or salt thereof is administered in the amount of about 200 mg/day. In some embodiments, the idasanutlin or salt thereof is administered in the amount of about 10 mg/m² body surface area to about 300 mg/m² body surface area per day. In some embodiments, the idasanutlin or salt thereof is administered in the amount of about 250 mg/day. In some embodiments, the idasanutlin or salt thereof is administered in the amount of about 300 mg/day. The amount may be any value or subrange within recited ranges, including endpoints. In some embodiments, the idasanutlin or salt thereof is administered via intravenous therapy. In some embodiments, the idasanutlin or salt thereof is administered orally.

In embodiments, the relugolix or salt thereof is administered in the amount of about 20 to about 360 mg/day. In some embodiments, the relugolix or salt thereof is administered in the amount of about 20 mg/day. In some embodiments, the relugolix or salt thereof is administered in the amount of about 40 mg/day. In some embodiments, the relugolix or salt thereof is administered in the amount of about 60 mg/day. In some embodiments, the relugolix or salt thereof is administered in the amount of about 80 mg/day. In some embodiments, the relugolix or salt thereof is administered in the amount of about 120 mg/day. In some embodiments, the relugolix or salt thereof is administered in the amount of about 160 mg/day. In some embodiments, the relugolix or salt thereof is administered in the amount of about 180 mg/day. In some embodiments, the relugolix or salt thereof is administered in the amount of about 360 mg/day. The amount may be any value or subrange within recited ranges, including endpoints. In some embodiments, the relugolix or salt thereof is administered orally.

In embodiments, one or more of the recited compounds (e.g., piperacillin, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea, cilengitide, (R,S)-ivosidenib, evacetrapib, idasanutlin, relugolix, hederacoside, cefoperazone, or a derivative or salt thereof) may be expressly excluded.

In embodiments, one or more additional agents may be administered to the subject. The one or more additional agents may treat SARS-CoV-2 infection, and/or a symptom of SARS-CoV-2 infection. In embodiments, the one or more agents include convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 or anti-SARS-CoV-2 vaccine. In embodiments, one or more of the recited agents may be expressly excluded.

In an aspect, a method of reducing infectivity of a SARS-CoV2 virus is provided. In embodiments, the method includes contacting the virus with at least one of: piperacillin, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea, cilengitide, (R,S)-ivosidenib, evacetrapib, idasanutlin, relugolix, hederacoside, cefoperazone, or a derivative or salt thereof. In embodiments, the virus is contacted with piperacillin, or derivative or salt thereof. In embodiments, the virus is contacted with N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea, or derivative or salt thereof. In embodiments, the virus is contacted with cilengitide, or derivative or salt thereof. In embodiments, the virus is contacted with (R,S)-ivosidenib, or derivative or salt thereof. In embodiments, the virus is contacted with evacetrapib, or derivative or salt thereof. In embodiments, the virus is contacted with idasanutlin, or derivative or salt thereof. In embodiments, the virus is contacted with relugolix, or derivative or salt thereof. In embodiments, the virus is contacted with hederacoside, or derivative or salt thereof. In embodiments, the virus is contacted with cefoperazone, or derivative or salt thereof. In embodiments, one or more of the recited compounds may be expressly excluded.

In an aspect, a method of reducing activity of SARS-CoV2 nsp5 protease is provided. In embodiments, the method includes contacting the nsp5 protease with at least one of: piperacillin, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea, cilengitide, (R,S)-ivosidenib, evacetrapib, idasanutlin, relugolix, hederacoside, cefoperazone, or a derivative thereof. In embodiments, the protease is contacted with piperacillin, or derivative or salt thereof. In embodiments, the protease is contacted with N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea, or derivative or salt thereof. In embodiments, the protease is contacted with cilengitide, or derivative or salt thereof. In embodiments, the protease is contacted with (R,S)-ivosidenib, or derivative or salt thereof. In embodiments, the protease is contacted with evacetrapib, or derivative or salt thereof. In embodiments, the protease is contacted with idasanutlin, or derivative or salt thereof. In embodiments, the protease is contacted with relugolix, or derivative or salt thereof. In embodiments, the protease is contacted with hederacoside, or derivative or salt thereof. In embodiments, the protease is contacted with cefoperazone, or derivative or salt thereof. In embodiments, one or more of the recited compounds may be expressly excluded.

The following experimental results are provided for purposes of illustration and are not intended to limit the scope of the invention.

### EXAMPLES

### EXAMPLE 1: In silico Screen of Compounds for Potential Interaction with Protease M^{pro}

Approximately 5000 compounds were screened by AMBER20 (D.A. Case, et al. (2020) AMBER 2020, University of California, San Francisco) for potential interaction with M^{pro}. A public database of compounds approved by the FDA or in phase 2/3 clinical trials was used (FDA-Approved Drug Library, cat. no. L1300 and Preclinical/Clinical Compound Library, cat. no. L3900, Selleckchem), as well as the published crystal structure of SARS-CoV-2 Mpro (RCSB Protein Databank Number 6LU7, Z. Jin, et al. Nature 582, 289-293 (2020)).

Compounds found to potentially interact by the AMBER20 analysis with M^{pro} were further evaluated using Autodock Vina (O. Trott and A.J. Olson, J. Comp. Chem. 31, 455-461 (2010)). Autodock Vina modeled the interaction of a compound with the protein and how the interaction may change the protein conformation to determine which compounds are most likely to bind to the M^{pro} protein. Compounds having a good score were further evaluated by visual inspection of the 3D compound-protein complex. Visual inspection evaluated numerous criteria, including formation of hydrogen bonds, conformation of the compound to the predicted binding site, and positioning of hydrophobic and hydrophilic constituents upon compound binding.

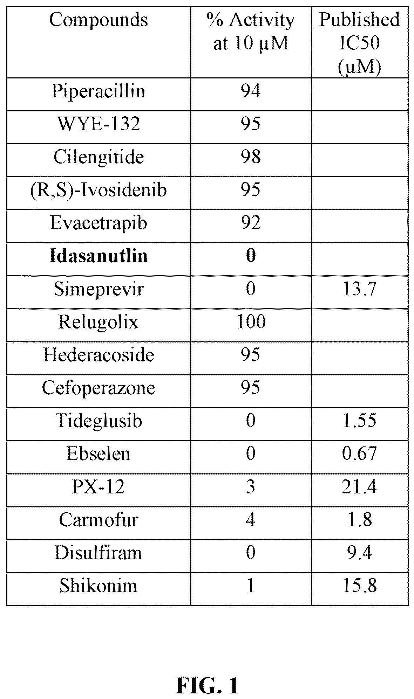

Finally, the remaining compounds were evaluated for molecular dynamics, e.g. how long the compound is expected to bind to M^{pro}. Resulting candidate compounds included piperacillin, WYE-132 (N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea), cilengitide, (R,S)-ivosidenib, evacetrapib, idasantulin, simeprevir, relugolix, hederacoside, and cefoperazone. See FIG. 1.

### EXAMPLE 2: FRET Assay To Determine M^{pro} Activity

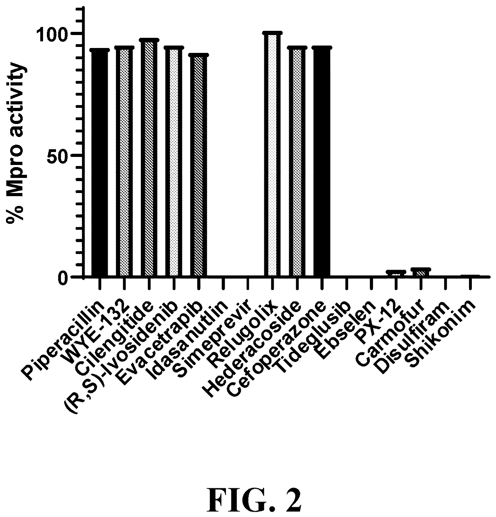

The compounds identified in Example 1 were analyzed by fluorescence resonance energy transfer (FRET) assay to determine their ability to bind and inhibit M^{pro} *in vitro.* The FRET assay conditions were adapted from Ma C, et al. Cell Research. (2020) 0:1-15. M^{pro} enzymatic activity was tested in the presence of the compounds listed in FIG. 1, and the results of the FRET activity assay are shown in FIG. 2.

All publications mentioned in this specification are herein incorporated by reference. Various modifications and variations of the described methods and system of the present invention will be apparent to those skilled in the art without departing from the scope and spirit of the present invention. Although the present invention has been described in connection with specific preferred embodiments, it should be understood that the invention as claimed should not be unduly limited to such specific embodiments. Indeed, various modifications of the described modes for carrying out the invention which are obvious to those skilled in biochemistry and biotechnology or related fields are intended to be within the scope of the claims.

### REFERENCES

1. C. Ma, et al. Boceprevir, GC-376, and calpain inhibitors II, XII inhibit SARS-CoV-2 viral replication by targeting the viral main protease. Cell Res. 0, 1-15 (2020).
2. Z. Jin, et al. Structure of Mpro from SARS-CoV-2 and discovery of its inhibitors. Nature 582, 289-293 (2020).
3. R. Salomon-Ferrer, D.A. Case, R.C. Walker. An overview of the Amber biomolecular simulation package. WIREs Comput. Mol. Sci. 3, 198-210 (2013).
4. D.A. Case, T.E. Cheatham, III, T. Darden, H. Gohlke, R. Luo, K.M. Merz, Jr., A. Onufriev, C. Simmerling, B. Wang and R. Woods. The Amber biomolecular simulation programs. J. Comp. Chem. 26, 1668-1688 (2005).
5. D.A. Case, K. Belfon, I.Y. Ben-Shalom, S.R. Brozell, D.S. Cerutti, T.E. Cheatham, III, V.W.D. Cruzeiro, T.A. Darden, R.E. Duke, G. Giambasu, M.K. Gilson, H. Gohlke, A.W. Goetz, R. Harris, S. Izadi, S.A. Izmailov, K. Kasavajhala, A. Kovalenko, R. Krasny, T. Kurtzman, T.S. Lee, S. LeGrand, P. Li, C. Lin, J. Liu, T. Luchko, R. Luo, V. Man, K.M. Merz, Y. Miao, O. Mikhailovskii, G. Monard, H. Nguyen, A. Onufriev, F.Pan, S. Pantano, R. Qi, D.R. Roe, A. Roitberg, C. Sagui, S. Schott-Verdugo, J. Shen, C.L. Simmerling, N.R.Skrynnikov, J. Smith, J. Swails, R.C. Walker, J. Wang, L. Wilson, R.M. Wolf, X. Wu, Y. Xiong, Y. Xue, D.M. York and P.A. Kollman. AMBER 2020, University of California, San Francisco (2020).
6. O. Trott, A. J. Olson. AutoDock Vina: improving the speed and accuracy of docking with a new scoring function, efficient optimization and multithreading. J. Comp. Chem. 31, 455-461 (2010).

### ASPECTS

1. A method of treating a SARS-CoV-2 virus infection in a subject in need thereof, the method comprising administering to the subject an effective amount of a pharmaceutical composition comprising a drug selected from the group consisting of piperacillin or a salt thereof, N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or a salt thereof, and cefoperazone or a salt thereof.
2. The method of aspect 1, wherein the piperacillin or salt thereof is administered in the amount of about 2 g to about 40 g powder per injection.
3. The method of aspect 2, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
4. The method of aspect 1, wherein the N-[4-[1-(1,4-dioxaspiro[4.5]dec-8-yl)-4-(8-oxa-3-azabicyclo[3.2.1]oct-3-yl)-1H-pyrazolo[3,4-d]pyrimidin-6-yl]phenyl]-N'-methylurea or salt thereof is administered in the amount of about 5 mg/kg to about 50 mg/kg.
5. The method of aspect 4, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
6. The method of aspect 1, wherein the cefoperazone or salt thereof is administered in the amount of about 1 g to about 12 g per day.
7. The method of aspect 6, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
8. A method of treating a SARS-CoV-2 virus infection in a subject in need thereof, the method comprising administering to the subject an effective amount of a pharmaceutical composition comprising a drug selected from the group consisting of cilengitide or a salt thereof and hederacoside or a salt thereof.
9. The method of aspect 8, wherein the cilengitide or salt thereof is administered in the amount of about 30 mg/m² to about 2400 mg/m² body surface area.
10. The method of aspect 9, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
11. The method of aspect 8, wherein the hederacoside or salt thereof is administered in the amount of about 35 mg/kg to about 50 mg/kg.
12. The method of aspect 11, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
13. A method of treating a SARS-CoV-2 virus infection in a subject in need thereof, the method comprising administering to the subject an effective amount of a pharmaceutical composition comprising a drug selected from the group consisting of (R,S)-ivosidenib or a salt thereof, evacetrapib or a salt thereof, idasanutlin or a salt thereof, and relugolix or a salt thereof.
14. The method of aspect 13, wherein the (R,S)-ivosidenib or salt thereof is administered in the amount of about 200 mg to about 1200 mg per day.
15. The method of aspect 14, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
16. The method of aspect 13, wherein the evacetrapib or salt thereof is administered in the amount of about 30 mg to about 600 mg per day.
17. The method of aspect 16, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
18. The method of aspect 13, wherein the idasanutlin or salt thereof is administered in the amount of about 120 to about 300 mg per day.
19. The method of aspect 18, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
20. The method of aspect 13, wherein the relugolix or salt thereof is administered in the amount of about 20 to about 360 mg per day.
21. The method of aspect 20, further comprising administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.

## Claims

1. A pharmaceutical composition for use in a method of inhibiting SARS-CoV-2 virus replication in a subject in need thereof, the method comprising administering to the subject an effective amount of the pharmaceutical composition, said pharmaceutical composition comprising a drug selected from the group consisting of piperacillin or a salt thereof, and cefoperazone or a salt thereof.

2. The pharmaceutical composition for use according to claim 1, wherein the piperacillin or salt thereof is administered in the amount of about 2 g to about 40 g powder per injection.

3. The pharmaceutical composition for use according to claim 2, wherein the method further comprises administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.

4. The pharmaceutical composition for use according to claim 1, wherein the cefoperazone or salt thereof is administered in the amount of about 1 g to about 12 g per day.

5. The pharmaceutical composition for use according to claim 4, wherein the method further comprises administering one or more agents selected from the group consisting of convalescent plasma, anti-SARS-CoV-2 antibodies or antibody-like molecules, tocilizumab, acalabrutinib, tofacitinib, ruxolitinib, baricitinib, anakinra, canakinumab, apremilast, mavrilimumab, sarilumab, VIR-7831, REGN-COV2, LY-CoV555, immune system modulators, interferons and interferon-like molecules, anticoagulants, prone positioning, antiviral therapeutics, remdesivir, lopinavir, ritonavir, oseltamivir, favipiravir, umifenovir, colchicine, ivermectin, antibacterial therapeutics, nitazoxanide, steroids, corticosteroids, dexamethasone, hydroxychloroquine, chloroquine, azithromycin, non-steroidal anti-inflammatories, calpain protease inhibitors, cathespin protease inhibitors, famotidine, nafamostat, camostat, ascorbic acid, zinc, RLF-100, tradipitant, LAU-7b, HFB30132A, AZD7442, CAP-1002, brilacidin, STI-1499, ebselen, disulfiram, tideglusib, carmofur, shikonin, PX-12, N3 and anti-SARS-CoV-2 vaccines.
